## Europäisches Patentamt
## European Patent Office
## Office européen des brevets

(11) Veröffentlichungsnummer: **0 193 588 B1**

# EUROPÄISCHE PATENTSCHRIFT
## veröffentlicht nach Art. 158 Abs. 3 EPÜ

(45) Veröffentlichungstag der Patentschrift: **03.07.91**

(51) Int. Cl.5: **A61L 27/00, A61K 6/06**

(21) Anmeldenummer: **85904613.8**

(22) Anmeldetag: **27.08.85**

(86) Internationale Anmeldenummer:
**PCT/EP85/00437**

(87) Internationale Veröffentlichungsnummer:
**WO 86/01726 (27.03.86 86/07)**

(54) **CARBONATAPATIT ENTHALTENDES MITTEL UND DIE VERWENDUNG VON CARBONATAPATIT FÜR IMPLANTATE.**

(30) Priorität: **10.09.84 DE 3433210**

(43) Veröffentlichungstag der Anmeldung:
**10.09.86 Patentblatt 86/37**

(45) Bekanntmachung des Hinweises auf die Patenterteilung:
**03.07.91 Patentblatt 91/27**

(84) Benannte Vertragsstaaten:
**AT BE CH DE FR GB IT LI LU NL SE**

(56) Entgegenhaltungen:
**EP-A- 0 016 906      EP-A- 0 058 867**
**EP-A- 0 104 640      EP-A- 0 120 689**
**DE-A- 2 821 354      DE-A- 3 038 047**
**FR-A- 2 223 325      US-A- 3 922 155**

(73) Patentinhaber: **Scheicher, Hans, Dr.med.**
**Dr.med.dent.**
**Rondell Neuwittelsbach 4**
**W-8000 München 19(DE)**

(72) Erfinder: **Scheicher, Hans, Dr.med.**
**Dr.med.dent.**
**Rondell Neuwittelsbach 4**
**W-8000 München 19(DE)**

(74) Vertreter: **Weisert, Annekäte, Dipl.-Ing.**
**Dr.-Ing. et al**
**Patentanwälte Kraus Weisert & Partner**
**Thomas-Wimmer-Ring 15**
**W-8000 München 22(DE)**

## Beschreibung

Die Erfindung betrifft ein Mittel zur Füllung von Knochen- und Zahndefekten, zum Knochenaufbau, für Knochenkontaktschichten und für den Knochen- und Zahnwurzelersatz, die Verwendung des Mittels sowie einen Implantatkörper.

Durch die schnelle Entwicklung der Chirurgie sind heute Operationen an Knochen und Gelenken möglich, die vor einiger Zeit noch undenkbar waren. Beispielsweise kann man heute Zysten, Knocheneiterherde und Malignome operativ aus Knochen entfernen. Dabei entstehen in dem Knochen Defekte, die ausgefüllt werden müssen, da sie durch die normalen Knochenreparaturvorgänge nicht mehr überbrückt werden können. Derartige Defekte können teilweise ein Volumen bis 600 cm³ aufweisen und müssen wieder gefüllt werden. Auch bei der Behandlung von Zähnen entstehen Hohlräume, die wieder gefüllt werden müssen.

Man verwendet zum Füllen derartiger Kavitäten Knochenersatzmaterialien in flüssiger, pastöser oder fester Form als Granulat oder Implantatkörper. Sind die zu füllenden Kavitäten nicht allzu groß, so sollen die Knochenersatzmaterialien die Hohlräume im Knochen vorübergehend ausfüllen und dem Körper ermöglichen, im Laufe der Zeit den Defekt selbst wieder mit lebendem Knochenmaterial zu überbrücken. Dabei kann das Knochenersatzmaterial entweder umwachsen werden und reizlos liegen bleiben oder vom lebenden Knochen langsam aufgelöst und ersetzt werden.

Bei der Füllung größerer Hohlräume mit Knochenersatzmaterial muß man ein mit den Knochen verträgliches Material verwenden. Als solche Materialen werden körpereigene oder körperfremde Knochenteile oder Granulate aus Hydroxylapatit eingesetzt. Das körpereigene Knochenmaterial steht nur in sehr begrenztem Umfang zur Verfügung, und zu seiner Gewinnung sind zusätzliche chirurgische Eingriffe erforderlich. Körperfremde, z.B. tierische, Knochenmaterialien müssen, um Abstoßungsreaktionen zu vermeiden, von allen Antigenen befreit werden, was in der Praxis nur teilweise gelingt.

Bei der Verwendung von Hydroxylapatit tritt eine Reizung des umliegenden Knochenmaterials auf. Es besteht daher ein Bedarf für ein Material, welches in flüssiger, pastöser oder fester Form zum Füllen von Kavitäten in Knochen, das heißt zur Füllung von Knochendefekten, verwendet werden kann.

Bei chirurgischen Eingriffen werden oft Knochenimplantate eingesetzt. Knochenimplantate sind Teile, die in die Knochen des Körpers eines Empfängers eingepflanzt werden und dauerhaft Skelettteile oder auch Zahnwurzeln ersetzen. Die äußere Schicht des Knochenimplantats, die mit dem lebenden Lagerknochen in Berührung kommt, wird als Knochenkontaktschicht bezeichnet. Als Knochenimplantate und als Knochenkontaktschichten sind derzeit Metalle, wie beispielsweise Edelstähle, Edelmetalle, Titan, keramische Materialien, wie beispielsweise Aluminiumoxid, Glaskeramik, Hydroxylapatitkeramik und Kunststoffe gebräuchlich.

Nach der Gewebeverträglichkeit werden diese Stoffe in biokompatible und bioaktive eingeteilt. Die biokompatiblen Stoffe werden vom Körper auf Dauer ohne Abstoßung toleriert. Die bioaktiven Stoffe wachsen wie körpereigenes Gewebe fest ein, wobei die chemische Zusammensetzung, die Oberflächenstruktur und die mechanischen Eigenschaften die Gewebeverträglichkeit bestimmen.

Die Metalle und manche Keramikmaterialien, wie beispielsweise Aluminiumoxidkeramik, sind biokompatibel. Im Körper findet stets eine Einscheidung durch Bindegewebe statt. Diese Bindegewebsschicht ermöglicht einen einigermaßen festen Halt des Implantats, jedoch keine kraftschlüssige Verbindung zum mineralischen Gerüst des Lagerknochens.

Wegen der fehlenden primären Integration in den Lagerknochen kann ein solches biokompatibles Implantat nur gering mechanisch belastet werden, da es sonst zu immer weiterer Verschlechterung des Haltes, verbunden mit Schmerzen und schließlich dem Verlust des Implantats, kommt. Dies zeigt sich beispielsweise bei den stets stark belasteten Hüftgelenksprothesen, bei denen heute schon mehr als ein Viertel der Operationen wegen der Lockerung eines früher eingesetzten Implantats durchgeführt wird.

Zur dauerhaften mechanischen Verankerung biokompatibler Implantate im Knochen sind daher zusätzlich Unterschneidungen, wie beispielsweise Gewinde, nötig. Ungeklärt ist bei allen metallischen Implantaten die Frage, ob diese giftige Metallionen an die Umgebung abgeben und so langfristig negative Wirkungen zeigen können.

Auch bei der Verwendung von Knochenzement kommt es trotz des zunächst besseren mechanischen Verbunds mit dem Lagerknochen mit einiger Verzögerung zu der geschilderten Lockerung.

Bei bioaktiven Materialien wächst das Knochenmaterial nach einiger Zeit direkt auf. Die besten Eigenschaften unter den bekannten Materialien zeigt hier Hydroxylapatit, der nach einer nur wenige Wochen dauernden Phase geringer Reizerscheinungen, die sich mikroskopisch durch Riesenzellen um das Implantat nachweisen lassen, ohne Zwischenschicht in den Lagerknochen integriert wird.

Bioaktive Materialien sind in der Regel schwer zu bearbeiten und mechanisch weniger stabil als die biokompatiblen Metalle oder Keramiken.

Man ist daher dazu übergegangen, kombinierte Implantate aus biokompatbilen Kernen, wie beispielsweise Titan, Edelstahl und Aluminiumoxid mit bioaktiven Oberflächenbeschichtungen zu verwenden (vgl. DE-PS 28 40 064). Eine solche kombinierte Implantatkonstruktion kann wesentliche Vorteile bringen, da hier hohe mechanische Festigkeit komplex geformter Implantate und schnelle und feste Verbindung mit dem Lagerknochen vereinigt werden. Es treten auch bei derartigen Implantaten mit den bisher bekannten bioaktiven Beschichten nach dem Einsetzen Reizerscheinungen auf, die jedoch nach einiger Zeit abklingen. Es besteht somit ein großer Bedarf nach einem Material, welches zur Herstellung von Implantaten oder zur Herstellung von Knochenkontaktschichten für Implantate verwendet werden kann und die erforderlichen bioaktiven und biomechanischen Eigenschaften aufweist.

Die folgenden Druckschriften befassen sich mit Carbonatapatiten in Bezug zum Knochenmineral und mit deren Einsatzmöglichkeiten für Implantate, wobei jedoch die tatsächlichen Einbaumechanismen für die Fremdionen an die entsprechenden Gitterplätze und insbesondere ihre Mengenverhältnisse nicht bekannt sind und sich die Angaben erheblich widersprechen:

In der EP-A-0 104 640 wird ein gesinterter Hochtemperatur-Apatit der Formel $Ca_{10}(PO_4)_6 Zm$ beschrieben, bei dem durch die Sinterung die Hydroxylionen im Gitter des Ausgangsmaterials dur Carbonationen ersetzt worden sind. Ähnliche Hochteperatur-Apatite werden in der US-A-3 922 155, in der DE-A-2 821 354 und in der DE-A 3 038 047 beschrieben. In allen Fällen fehlt die Angabe einer Alkali-Komponente und durch die Sinterung ist eine gravierende Änderung der Materialeigenschaften eingetreten. In der EP-A-0 016 906 wird die Herstellung eines carbonathaltigen Biomaterials durch hydrothermale Umwandlung von Calcit oder Aragonit biogenen Ursprungs beschrieben. Bei diesem Verfahren kann der Carbonatgehalt des Materials und dessen Position im Gitter nicht gesteuert werden. Zudem ist durch die Wahl des Verfahrens die Entstehung eines Phasengemisches aus carbonathaltigem Apatit und mehr oder weniger großen Anteilen von Whitlockit vorprogrammiert. In der EP-A-0 120 689 sind Hochtemperatur-Sinterkeramiken angegeben, die Hydroxylapatit, Tricalciumphosphat und/oder Tetracalciumphophat als Phasengemenge enthalten und denen Zusatzstoffe, wie Medikamente, beigegeben werden können.

Der vorliegenden Erfindung liegt die Aufgabe zugrunde, ein Mittel zur Füllung von Knochendefekten, zum Knochenaufbau, für den Knochen- und Zahnwurzelersatz und für Knochenkontaktschichten zur Verfügung zu stellen, welches ohne oder mit nur äußerst geringen Reizerscheinungen schnell und fest mit dem Knochen verwächst.

Erfindungsgemäß soll ein Mittel zur Verfügung gestellt werden, welches, wenn es als Füllung von Knochendefekten, zum Knochenaufbau und als Knochen- und Zahnwurzelersatz verwendet wird, keine Abstoßungsreaktionen ergibt und das unbegrenzt zur Verfügung steht. Das Mittel soll ohne Reizphase einwachsen und den lebenden Knochen ersetzen bzw. durch lebende Knochen ersetzt werden.

Erfindungsgemäß soll ein Mittel zur Verfügung gestellt werden, bei dem eine Verkürzung der beobachteten Reizphase und eine Beschleunigung der Knocheneinlagerung möglich ist, wenn es als Knochenkontaktschicht verwendet wird, so daß bioaktive Implantete mit höchst möglicher Erfolgsquote für den Patienten eingesetzt werden können.

Eine wesentlich verkürzte Einheilphase bedeutet für den Patienten, daß er die entsprechenden Körperteile früher mechanisch belasten kann, wodurch auch das Operationsrisiko vermindert wird.

Gegenstand der Erfindung ist ein Mittel zur Füllung von Knochen- und Zahndefekten, zum Knochenaufbau, für Knochenkontaktschichten und für Knochen- und Zahnwurzelersatz, das dadurch gekennzeichnet ist, daß es Carbonatapatit der Formel I

$$Ca_{10-a-b-x} Me_a^{II} Me_b^{I} (PO_4)_{6-x} (CO_3)x (OH)_{2-b-x}(H_2O)_w \quad (I)$$

worin

a   die Anzahl der zweiwertigen Metallionen außer Ca bedeutet und für einen Wert von 0 bis 4,0 steht,

b   die Anzahl der einwertigen Metallionen bedeutet und für einen Wert von 0,05 bis 1,0 steht,

w   die Anzahl der in das Kristallgitter integrierten Wassermoleküle bedeutet und für 0 bis 1,8 steht,

x   die Anzahl der Carbonationen bedeutet und für einen Wert von 0,2 bis 2 steht,

gegebenenfalls zusammen mit körperverträglichen Zusatzstoffen und/oder Verdünnungsmitteln enthält.

Die körperverträglichen Zusatzstoffe können organische und/oder anorganische Bindemittel, physiologische Salzlösungen und/oder Medikamente und/oder Hormone sein. Das Mittel kann 1 bis 100 Gew.-% Carbonatapatit enthalten. Es kann weiterhin Carbonatapatit der Formel

$$C_{10-x}(PO_4)_{6-x}(CO_3)_x(OH)_{2-x}$$

worin X für 0,2 bis 2 steht, enthalten.

Gegenstand der Erfindung ist weiterhin die Verwendung des Carbonatapatits der obigen Formel I zur Füllung von Knochen-und Zahndefekten, zum Knochenaufbau, für Knochenkontaktschichten, als Knochenersatz und als Zahnwurzelersatz.

Gegenstand der Erfindung ist auch ein Implantatkörper, der dadurch gekennzeichnet ist, daß er vollständig oder teilweise aus Carbonatapatit besteht oder daß er aus einem für Implantatkörper bekannten Material besteht und vollständig oder teilweise mit einer Schicht aus Carbonatapatit der obigen Formel I überzogen ist.

Überraschenderweise wurde gefunden, daß die beanspruchten Carbonatapatite oder carbonathaltigen Apatite mit dem Knochen im Körper eine feste Bindung eingehen, ohne daß eine Phase langer Reizerscheinungen auftritt. Diese Carbonatapatite zeigen für das Aufwachsen von Knochenmineral im Körper verbesserte Eigenschaften im Vergleich mit Hydroxylapatit. Dies ist vermutlich auf ihre chemischen und kristallstrukturellen Eigenschaften zurückzuführen. Im Gegensatz zu dem oben erwähnten Hochtemperatur-Apatiten handelt es sich um gefällte Carbonatapatite, bei denen ein Teil der Phosphationen durch Carbonationen ersetzt sind.

Die angestellten Untersuchungen lassen den Schluß zu, obgleich dies keine Beschränkung auf irgendeine Theorie sein soll, daß die anfänglichen Reizerscheinungen und das verzögerte Einwachsen von Hydroxylapatit im Knochen als eine chemische Umwandlung der Implantatoberfläche zu deuten ist. Hydroxylapatit wird im carbonathaltigen Milieu des Knochens vermutlich oberflächlich carbonatisiert, das heißt in Carbonatapatit umgewandelt, bevor eine Ablagerung von Knochenmineral am Implantat erfolgen kann. Durch das erfindungsgemäße Material wird diese Phase übersprungen. Ein Implantat mit einer Oberfläche aus Carbonatapatit kann also schneller und fester im Knochen einwachsen. Die Gitterparameter des Carbonatapatits weichen von denen des Hydroxylapatits ab. Ein epitaktisches Aufwachsen von Knochenmineral auf das Implantat und damit auch eine feste kristalline Verbindung wird also gefördert, wenn das Implantat als Wirtsgitter schon Carbonatapatit-Kristalle bietet.

Bei Tierversuchen, in denen Implantate mit Carbonatapatit-Oberflächen im Femur von Ratten eingesetzt wurden, konnte schon nach einer Woche eine feste Verbindung zum Lagerknochen beobachtet werden. Dies war überraschend und hat nicht nahegelegen. Das rasche Anwachsen des Implantats ist auch für den langfristigen Erfolg von Bedeutung, da schon geringe Bewegungen bei mechanischer Belastung zu einer Umkleidung des Implantats mit Bindegewebe führen, die ein direktes Aufwachsen von Knochenmaterial dauerhaft verhindert. Durch eine Oberflächenbeschichtung mit dem erfindungsgemäßen Material wird damit eine frühere Belastbarkeit und eine bessere Prognose des Implantats gegenüber Implantaten aus den bisher üblichen Materialien gewährleistet.

Der erfindungsgemäß verwendete carbonatapatithaltige Apatit, der vereinfacht als Carbonatapatit bezeichnet wird, ist ein komplexes Calcium-Phosphat-Carbonat-Salz, mit einer chemischen Struktur analog der von Hydroxylapatit.

Der Carbonatapatit kann auch Hydroxylapatit enthalten. Wesentlich ist, daß der Gehalt an Carbonationen gegenüber der chemischen Stammsubstanz aller Apatite, dem Hydroxylapatit, die funktionell wesentliche und quantitativ größte Abweichung bei dem erfindungsgemäßen Material darstellt. Aus technischen Gründen sind chemisch ganz reine Carbonatapatite nicht herstellbar. Geringe Verunreinigung mit anderen Ionen, insbesondere Hydrogenphosphat, sind unvermeidlich und zum Teil auch, wegen der angestrebten Ähnlichkeit mit dem Knochenmineral, wünschenswert.

Der Begriff Carbonatapatit ist auch in der wissenschaftlichen Literatur nicht einheitlich. Im folgenden werden daher die chemischen Formeln der Kristalleinheitszelle verschiedener Apatite aufgeführt:

I. Hydroxylapatit:

$$Ca_{10} (PO_4)_6 (OH)_2$$

II. Carbonatapatit:

$$Ca_{10-x} (PO_4)_{6-x} (CO_3)_x (OH)_{2-x}$$

wobei gilt: $0 < x \leq 2$

Der Wert x bestimmt den Grad der Carbonatisierung, dieser soll etwa dem Carbonatgehalt des natürlichen Knochenminerals entsprechen, dessen Zusammensetzung angenähert

$$Ca_{8,5} Na_{0,25} (PO_4)_{4,8} (HPO_4)_{0,2} (CO_3)_{1,0} (OH)_{0,5}$$

lautet. Der Wert von x liegt zwischen 0,2 und 2, vorzugsweise zwischen 0,6 und 1,6, besonders bevorzugt zwischen 0,9 und 1,2.

III. Carbonatapatit:

$$Ca_{10-x-y}(PO_4)_{6-x-y}(HPO_4)_y (CO_3)_x (OH)_{2-x-y}$$

worin x die Anzahl der Carbonationen bedeutet und den oben unter I. angegebenen Wert besitzt und y die Anzahl der Hydrogenphosphationen bedeutet und einen Wert von 0 bis 1,8, vorzugsweise 0,05 bis 0,8, besitzt.

IV.

$$Ca_{10-a-b-0,5w-x-y}Me_a^{II} Me_b^{I} (PO_4)_{6-x-y}(HPO_4)_y (Hal)_z (CO_3)_x (OH)_{2-b-w-x-y-z}(H_2O)_w \cdot (H_2O)$$

Carbonathaltige Apatitstruktur mit den häufigsten und wichtigsten Fremdionen, wobei Hal für Halogene (Fluor, Chlor, Brom und Jod, vorzugsweise Fluor und Chlor, besonders bevorzugt Fluor) steht und Me für Metallionen steht. Bedeutung hat besonders Na$^+$ als einwertiges Metallion und F$^-$ zur Verbesserung der Langzeitstabilität. Die in Formel IV der Vollständigkeit halber angegebene Adsorption von Wassermolekülen ist bei allen Apatitstrukturen möglich und wird im allgemeinen bei der chemischen Beschreibung der Kristalleinheitszelle außer Betracht gelassen.

a　bedeutet die Anzahl der zweiwertigen Metallionen und steht für 0 bis 9,8, vorzugsweise 0 bis 4,0, besonders bevorzugt für 0 bis 0,6,

b　bedeutet die Anzahl der einwertigen Metallionen und steht für 0 bis 1,8, vorzugsweise 0 bis 1,0, besonders bevorzugt für 0,05 bis 0,5,

w　bedeutet die Anzahl der in das Kristallgitter integrierten Wassermoleküle und steht für 0 bis 1,8, bevorzugt für 0 bis 0,6,

x　bedeutet die Anzahl der Carbonationen und steht für 0,2 bis 2, vorzugsweise für die unter II. angegebenen Werte,

y　bedeutet die Anzahl der Hydrogenphosphationen und steht für 0 bis 1,8, vorzugsweise 0 bis 0,5,

z　bedeutet die Anzahl der Halogenidionen und steht für einen Wert von 0 bis 1,8.

Beispiele für einwertige Metallionen sind Alkalimetalle und Silber. Beispiele für zweiwertige Metallionen sind Mg$^{2+}$, Pb$^{2+}$ und Sr$^{2+}$.

Bei einem im folgenden erläuterten Verfahren zur Darstellung des Carbonatapatits fällt ein Produkt an, welches aus Kügelchen oder Perlen besteht, die im Inneren aus Hydroxylapatit bestehen und außen mit einer dünnen Carbonatapatitschicht überzogen sind. Ein derartiges Produkt ist erfindungsgemäß besonders gut geeignet. Wenn in der vorliegenden Anmeldung von Carbonatapatit gesprochen wird, sollen daher darunter Produkte verstanden werden, wie sie oben unter II., III. und IV. aufgeführt wurden.

Das erfindungsgemäße Carbonatapatit enthaltende Mittel kann gegebenenfalls körperverträgliche Zusatzstoffe und/oder Verdünnungsmittel, physiologische Salzlösungen und/oder Medikamente und/oder Hormone enthalten. Der Gehelt an diesen Stoffen richtet sich jeweils nach der Anwendung, und die Verwendung dieser Stoffe ist dem Fachmann geläufig. Man kann all diese körperverträglichen Zusatzstoffe und Verdünnungsmittel verwenden, wie sie bereits jetzt auf den genannten Gebieten eingesetzt werden.

Beispiele für organische Bindemittel sind Acrylate, wie Polymethylmethacrylatknochenzemente, andere polymerisierbare Kunststoffe, wie Polyester, gelierfähige Polysaccharide, wie Agarose, Gelatine, vernetzende Polypeptide, wie Fibrinkleber.

Beispiele für anorganische Bindemittel sind Silicate, wie Gläser, keramische Massen, hydraulische Bindemittel, wie Zemente, sowie Silicone.

Als Beispiele für Salzlösungen können Kochsalzlösung, Ringerlösung und Bicarbonatlösung, als Beispiele für Medikamente Antibiotika, Carnosin, Heparin, Hyaluronidase, kolloidales Silber oder ein Silbersalz und als Beispiel für Hormone anabole Steroide, Calcitonin, Parathormon und Wachstumshormon (STH) genannt werden.

Das erfindungsgemäße Mittel kann je nach der Anwendung 1 bis 100 Gew.-% Carbonatapatit enthalten. Das oben erwähnte Produkt, welches aus Hydroxylapatit mit einer Haut aus Carbonatapatit besteht und das erfindungsgemäß bevorzugt bei bestimmten Anwendungen, wie sie im folgenden erläutert werden, eingesetzt wird, enthält beispeilsweise nur sehr wenig Carbonatapatit. Enthält das erfindungsgemäße Mittel 100% Carbonatapatit, so wird es als solches verwendet, oder es kann vom Arzt, beispielweise dem Chirurgen, mit Eigenblut oder wäßrigen Lösungen verdünnt und dann eingesetzt werden. Das erfindungsgemäße Mittel kann in flüssiger oder fester Form vorliegen, und der Zusammensetzung sind keinerlei Grenzen gesetzt.

Die genaue Zusammensetzung des erfindungsgemäßen Mittels richtet sich nach der beabsichtigten Verwendung und kann vom Fachmann leicht festgelegt werden.

Das erfindungsgemäße Mittel kann in flüssiger Form als Suspension oder Dispersion, in pastöser Form oder in fester Form, in Form eines Granulats oder als feines Pulver vorliegen.

Enthält das erfindungsgemäße Mittel nahe zu reinen Carbonatapatit, der gegebenenfalls, wie oben ausgeführt, Hydroxylapatit enthalten kann, so kann dieser als solcher zur Oberflächenbeschichtung von Implantaten verwendet werden, oder er kann beispielsweise mit Eigenblut oder physiologischer Salzlösung oder mit sterilem Wasser vor der Anwendung vermischt und dann eingesetzt werden. Liegt das erfindungsgemäße Mittel in flüssiger oder pastöser Form vor, so kann es mit Hilfe von Spritzen etc., wie es dem Fachmann geläufig ist, eingesetzt werden.

Die Herstellung des carbonathaltigen Apatits kann beispielsweise nach einem der folgenden Verfahren erfolgen.

1. Carbonathaltigen Apatit kann durch Ionenaustausch aus Hydroxylapatit in carbonathaltigen wäßrigen Suspensionen gewonnen werden.

Man verwendet hierbei Suspensionen von synthetischem oder aus geglühtem Knochen gewonnenen Hydroxylapatitpulver. Die Geschwindigkeit der Carbonatisierung nimmt mit der Temperatur und der Carbonatkonzentration der Lösung zu. Das Verfahren wird daher bevorzugt in heißen Lösungen mit hohem Carbonatgehalt, beispielsweise bei Reaktionstemperaturen von 20 bis 100°C, vorzugsweise bei 80 bis 100°C,durchgeführt. Das Carbonat wird vorzugsweise in Form der Kalium- und/oder Natriumsalze (als Carbonat oder Bicarbonat) in die Lösung eingebracht.

Als Ausgangsmaterial zur Gewinnung des Carbonatapatits wird bevorzugt sehr feinkristalliner Hydroxylapatit mit hoher spezifischer Oberfläche, vorzugsweise feinkristalliner synthetischer Hydroxylapatit oder Hydroxylapatit aus tierischem Material,wie er z.B. nach dem in der DE-PS 28 40 064 beschriebenen Verfahren gewonnen wird, eingesetzt.

Vorzugsweise wird bei diesem Verfahren gerührt. Die Reaktionsdauer hängt von der verwendeten Temperatur und Konzentration der Carbonatlösung ab und liegt im Bereich von 10 Minuten bis 24 Stunden.
Bei der Verwendung dieser Ausgangsmaterialien ist jedoch immer mit mehr oder weniger großen verbleibenden Kernen aus Hydroxylapatit zu rechnen. Man erhält ein Material aus Kristallen mit einer Außenschichtstärke von nur ca. 0,5 bis 2 nm aus Carbonatapatit und verbleibenden Kernen aus Hydroxylapatit. Homogener Carbonatapatit ist nach diesem Verfahren nicht herstellbar. Das bei diesem Verfahren erhaltene Produkt ist erfindungsgemäß besonders für Knochenkontaktschichten bevorzugt.
2. Homogene Carbonatapatit-Kristalle können direkt aus Calcium-Phosphat-Carbonat-Lösungen ausgefällt werden.

Dabei gibt man zu siedenden wäßrigen carbonat- und phosphathaltigen Lösungen mit dem gewünschten Carbonat/Phosphat-Verhältnis Calcium, z.B. in Form seines Nitrats, hinzu. Das Verhältnis Carbonat/Phosphat muß in der Lösung höher sein als das im Kristall gewünschte. Der höchstmögliche Quotient 0,5 im Kristall wird bei 2:1 in der Lösung erreicht. Konzentration 0,01 - 10,0 molar.

Vorzugsweise erfolgt die Zugabe des Calciumsalzes tropfenweise, indem man beispielsweise eine wäßrige Calciumnitratlösung hinzutropft. Das Carbonat und das Phosphat werden vorzugsweise in Form ihrer Kalium- und/oder Natriumsalze in die Ausgangslösung eingebracht. Um den Einfluß von Hydrogenphosphationen gering zu halten, soll die Reaktion im stark alkalischen Bereich, z.B. bei einem pH im Bereich von 12 bis 14, vorzugsweise bei pH 13,stattfinden.

Nach dem Zusetzen des Calciumnitrats läßt man die Lösung zur Ausfällung einige Stunden bei 20 bis 100°C, vorzugsweise bei 80 bis 100°C,stehen. Die entstehenden Carbonatapatitkristalle werden dann abfiltriert, mit destilliertem Wasser gewaschen und in der Luft getrocknet.

Das bei diesem Verfahren erhaltene Produkt ist erfindungsgemäß für die Füllung von Knochendefekten bevorzugt.
3. Carbonatapatit kann auch durch Umsetzung von Hydroxylapatit in Pulverform mit Kohlendioxidgas hergestellt werden.

Bei diesem Verfahren wird Hydroxylapatit in Pulver-, Granulat- oder Festkörperform mit Kohlendioxidgas bei hoher Temperatur,beispielsweise bei 200 bis 1000°C, vorzugsweise 400 bis 800°C,und bei hohem Druck von 10 bis 100 atü, vorzugsweise 15 bis 40 atü, einige Stunden bis mehrere Tage carbonatisiert. Die Reaktionszeit hängt von dem gewünschten Carbonatisierungsgrad ab. Dann wird in einer Kohlenstoffatmosphäre schnell auf unter 200°C abgekühlt, z.B. durch Einfüllen von flüssigem Kohlenstoffdioxidgas. Die genauen Reaktionsbedingungen können vom Fachmann leicht bestimmt werden. Dieses Verfahren eignet sich insbesondere zur Herstellung von großen Mengen Carbonatapatit.

Anhand der beigefügten Zeichnungen wird die Erfindung näher erläutert.

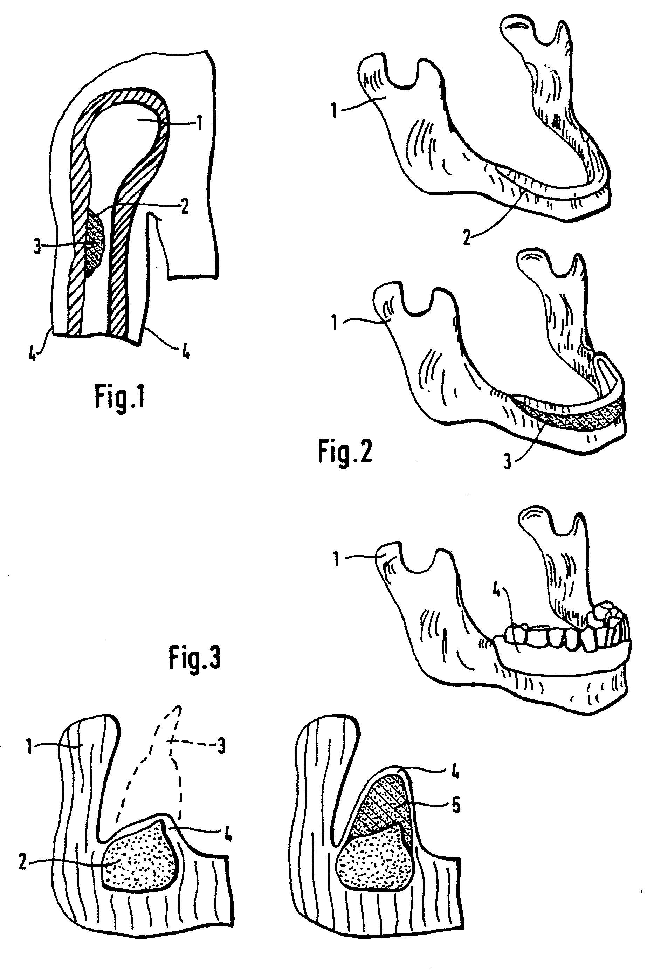

Figur 1 zeigt einen Knochen 1 mit einem Knochendefekt 2, der beispielsweise durch eine Operation bei der Entfernung einer bösartigen Geschwulst entstanden ist. In den Hohlraum 2 wird das erfindungsgemäße Material 3 eingefüllt und wächst dort fest. Der Knochen ist nach dem Festwachsen, was praktisch ohne Reizerscheinungen geschieht, wieder normal belastbar und verwendungsfähig, ohne daß der Patient Schmerzen hat. Gemäß dem in Figur 1 dargestellten Verfahren können auch Riesendefekte, beispielsweise Hohlräume mit einem Inhalt von 200 bis 800 cm³, ausgefüllt werden.

In Figur 2 ist eine andere Einsatzmöglichkeit des Carbonatapatits dargestellt. Gemäß Figur 2 kann die äußere Form eines Knochens abgeändert werden, indem man beispielsweise einen geschwundenen, zahnlosen Kieferknochen durchtrennt und nach dem sogenannten Sandwich-Verfahren mit einer Schicht Carbonatapatit unterfüttert. In Figur 2 bedeuten 1 den Knochen, 2 die Trennlinie und 3 das erfindungsgemäße Material. 4 ist eine aufgesetzte Vollprothese.

Die in Figur 3 dargestellte Ausführungsform dient insbesondere zum Aufbau von Knochen, beispielsweise bei Schwund von Kieferkämmen, oder in der plastischen Chirurgie zum Formen von Gesichtsknochen nach Verletzung oder Tumoroperation. Man kann in solchen Fällen die Knochenhaut lösen, eine Tasche bilden, in die gebildete Umhüllung den Carbonatapatit füllen und dadurch den Knochen aufbauen.

Figur 3 zeigt einen sagittalen Schnitt durch den Unterkiefer im Frontzahnbereich, dabei bedeuten 1 die Unterlippe, 2 den Knochen, 3 die ursprüngliche Kieferkontur mit Zahn, 4 die Schleimhaut und 5 das erfindungsgemäße Material.

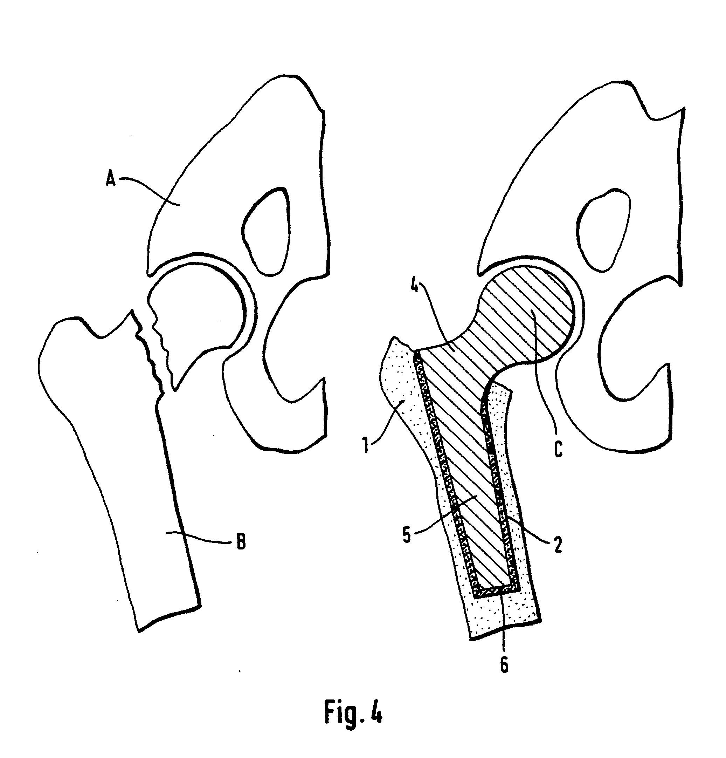

Wie in Figur 4 dargestellt, ist es weiterhin möglich, in den Knochen 1, der einen Hohlraum 2 aufweist, einen Implantatkörper 4 einzusetzen. Der Implantatkörper 4 besteht aus einem Kern 5 und einer Knochenkontaktschicht 6 aus Carbonatapatit. Der Implantatkörper 4 ist an seiner Oberfläche mit Carbonatapatit beschichtet. Die Implantatkörper der Figuren 1,2,3 und 4 können aber auch vollständig aus Carbonatapatit bestehen.

In Figur 4 ist der erfindungsgemäße Einsatz von Carbonatapatit anhand einer Hüftgelenksendoprothese näher erläutert. Ein Schenkelhalsbruch mit ungünstig verlaufender Bruchlinie (Hüftknochen A, Schenkelknochen B) erfordert die chirurgische Versorgung mit einem künstlichen Gelenkkopf C. Die Verbindung zum Lagerknochen erfolgt über ein Implantat, welches mit Carbonatapatit gemäß der vorliegenden Erfindung beschichtet ist.

Bei einer solchen Ausführungsform findet ein rascher, fester und dauerhafter Verbund zwischen Endoprothese und Knochen statt, der ausschlaggebend ist für den Erfolg des Eingriffs. Hüftgelenksoperationen sind für die Patienten sehr belastend. Das derzeit übliche Einkleben des Implantats mit Knochenzement ist verbesserungsbedürftig, und heute sind bereits, wie schon ausgeführt wurde, ein Viertel aller derartigen Hüftgelenkseingriffe Wiederholungsoperationen, die wegen der Lockerung der Endoprothese notwendig werden. Die überraschenderweise gefundene Verkürzung der Reizphase läßt eine Verminderung der Häufigkeit solcher Zweitoperationen erwarten.

In Figur 5 ist der zeitliche Verlauf des Einbaus von Carbonationen in Hydroxylapatit in Abhängigkeit von der Carbonatkonzentration und der spezifischen Oberfläche des Apatitpulvers dargestellt. In Figur 6 ist das IR-Spektrum des Carbonatapatits von dem gemäß Beispiel 3 erhaltenen Produkt dargestellt, und in Figur 7 ist das IR-Spektrum vom Säugetierknochen dargestellt.

Im folgenden wird der Einsatz des erfindungsgemäßen Mittels und die Verwendung des Carbonatapatits näher erläutert.

1. Verwendung von Carbonatapatit zur Füllung von Knochen- und Zahndefekten sowie zum Knochenaufbau.

1.1 Lose Füllung

Carbonatapatit wird als lose Füllung in den Fällen verwendet, bei denen die Füllung nach der Einbringung keine mechanische Festigkeit haben muß, keinen primären Abschluß des Defekts gegen die Umgebung bilden muß sowie keinen oder nur geringen verformenden Kräften ausgesetzt ist und vorwiegend eine rasche Heilung bewirken soll.

Funktion

Das Mittel soll als vorübergehender Platzhalter im Defekt dienen und möglichst schnell von lebendem Knochen durchwachsen sowie später von diesem abgebaut und ersetzt werden. Wesentlich ist, daß das Einwachsen von normalem Narbengewebe verhindert wird und die einwachsenden Knochenzellen ein günstiges Milieu, wie ausreichende Porosität, physiologische Zusammensetzung sowie knochenmineralähnliche feste Partikel, vorfinden, welche durch neu angelagertes Knochenmineral nur noch verbunden werden müssen, um den Defekt knöchern zu überbrücken.

Je nach Lage und Größe des Defekts wird so die Wiederherstellung normal belastbaren Knochens überhaupt erst ermöglicht oder dramatisch von Jahren auf Wochen verkürzt.

Beispiele

Als Beispiel hierfür dienen die Auffüllung kleinerer Defekte, bei denen der verbleibende Knochen tragfähig ist oder durch andere Maßnahmen, wie Verplattung, Gipsverband, gehalten wird, wie Knochenzysten im Kiefer, Zahnfach nach Extraktion, paradontale Knochendefekte, ausgeräumte Knocheneiterherde (Osteomyelitis), Tumorbett im Knochen, Defekte bei Trümmerbruch (chirurgische Verbindung der Knochenteile mit Plattenosteosynthese) oder Knochenaufbau in "Sandwichtechnik", wie es beispielsweise in Figur 2 dargestellt ist.

Zu diesem Zweck wird ein Pulver oder Granulat von Carbonatapatit mit einer Partikelgröße von etwa 0,1 bis 2 mm verwendet. Das Mittel kann als Zusatzstoff für wäßrige Medien, wie physiologische Puffer und Salzlösungen oder Eigenblut, und eventuell relativ weiche resorbierbare Bindemittel, wie Polysaccharide oder Polypeptide, enthalten. Der Zusatz muß ausreichen, um ein gasfreies Gemisch zu erzielen, ein geringer Überschuß ist zur leichteren Verarbeitung evtl. wünschenswert. Der Carbonatapatit besitzt beispielsweise eine rechnerische Dichte von 3,4 $g/cm^3$, eine Schüttdichte nach Partikelform und -größe von 1,0 $g/cm^3$ bis 3,0 $g/cm^3$ (Zusatzstoffe Dichte um 1,0 $g/cm^3$).

Ein Mittel für diese Anwendung enthält beispielsweise 30 bis 80 Gew.-% Carbonatapatit und 70 bis 20 Gew.-% Zusatzstoffe der oben aufgeführten Art.

Als weitere Zusätze in geringer Menge kommen beispielsweise infektionshemmende, durchblutungsfördernde und knochenwachstumsfördernde Medikamente, Hormone und Chemikalien in Betracht.

Für den tatsächlichen Gebrauch kann das Mittel steril, für kleinere Mengen in Spritzen, sonst in Schraubgläsern, Dosen etc. abgepackt werden. Zur Anwendung mit Blut oder Zusatzstoffen kann man auch das Mittel und das Zusatzmittel getrennt in fester Form abpacken, und diese werden dann beim Gebrauch miteinander vermischt.

1.2 Feste Füllungen

Feste Füllungen werden bei den Fällen verwendet, in denen das Mittel nach dem Ein- oder Aufbringen zu einer festen, mechanisch belastbaren und formstabilen Masse erstarren muß, einen primär dichten Abschluß des Defekts bilden muß oder fest am Knochen anhaften muß.

Funktion

Das Mittel soll den Defekt sofort fest und randschlüssig ausfüllen, mit dem Knochen schnell fest verwachsen und im Laufe der Zeit langsam von lebendem Knochen durchwachsen und teilweise ersetzt werden. Wichtig ist je nach Anwendungsgebiet der dichte Abschluß, der das Eindringen von Keimen verhindert, die kraftschlüssige Verbindung zum Knochen bei mechanischer Belastbarkeit oder die stabile Form und feste Anhaftung. Das Bindemittel bildet eine stabile Matrix, in der die Carbonatapatitpartikel festgehalten werden, und haftet nach dem Abbinden am Knochen an. Die Carbonatapatitpartikel an der Oberfläche erlauben ein Anwachsen der Füllung im Knochen, bei ausreichender Porosität wird das Material von lebendem Knochen durchwachsen, eine bleibende, nicht resorbierbare Bindemittelmatrix kann dabei eventuell in Kauf genommen werden.

Das Mittel ermöglicht einen funktionell sofort belastbaren Ersatz von Knochenteilen, der langsam wieder von lebendem Knochen ersetzt oder wenigstens durchsetzt wird.

Beispiele

Beispiele für die Anwendung von festen Füllungen sind das Auffüllen großer Knochendefekte als Infektions-, Verletzungs- oder Tumorfolge, bei denen der verbleibende Knochen nicht mehr ausreichend belastbar ist, Verkitten der Fragmente bei Trümmerbrüchen, freier Knochenaufbau (vgl. Figur 3) sowie Füllungen des Nervkanals im Zahn.

Zusammensetzung des Mittels

Für diesen Anwendungszweck wird ein Pulver oder ein Granulat oder Teile aus Carbonatapatit (feine Pulver für Zahnfüllungen, mittlere Körnungen 0,1 mm für Knochendefekte, Granulate um 1 mm zum Knochenaufbau) und Teile zum Knochenersatz verwendet. Als Zusatzstoffe sind bei Körpertemperatur aushärtende, wenig exotherme, ungiftige Bindemittel, wie Acrylate, Polyester, Zemente usw., geeignet, bei sehr geringer Belastung sind eventuell auch Geliermittel geeignet.

Das Mittel kann in Form eines Zweikomponenten-Mittels in den Handel gebracht werden. Die eine Komponente besteht aus Carbonatapatit und die zweite Komponente besteht aus dem Bindemittel. Man kann aber auch fertige Mischungen aus Carbonatapatit und Bindemittel herstellen, die gegebenenfalls weitere Zusatzstoffe enthalten, und diese wie bei 1.1 beschrieben in den Handel bringen. Als Zusatzstoffe können die oben erwähnten Zusatzstoffe sowie zusätzlich Lösungs- und/oder Treibmittel zur Erhöhung der Porosität mit verwendet werden.

2. Beschichten und Herstellen von Implantaten mit Carbonatapatit

2.1 Knochenkontaktschichten

Soll ein Knochenimplantat die guten mechanischen Eigenschaften biokompatibler Materialien mit den idealen bioaktiven Kontakteigenschaften des Carbonatapatits vereinen, ist eine Beschichtung sinnvoll.

Funktion

Die Knochenkontaktschicht auf dem Implantat soll dem Lagerknochen eine möglichst knochenähnliche Oberfläche bieten, so daß das Implantat nicht als Fremdkörper erkannt und wie ein frakturiertes Knochenteil fest in das Mineralgerüst des Knochens eingebaut wird. Dabei kommt es nicht auf die Dicke der Kontaktschicht, sondern nur auf die chemische Zusammensetzung der äußersten Molekülschicht an. Wichtig ist weiter die feste Anhaftung an dem Implantatkern. Eine solche Kontaktschicht ermöglicht eine sich schnell bildende, kraftschlüssige Verbindung zwischen Knochen und Implantat, damit werden die Einheilzeiten verkürzt und Lockerungen weniger wahrscheinlich.

Beispiele

Jede Art von Knochenimplantaten, einschließlich von Zahnwurzelimplantaten, können mit Knochenkontaktschichten versehen werden. Es ist möglich, ein trockenes Pulver aus Carbonatapatit auf Implantate aus anderen Materialien aufzupressen.

Man kann weiterhin Gemische aus Carbonatapatit mit den oben unter 1.2 beschriebenen Zusatzstoffen auf Implantate aufbringen. Man kann auch den Implantatkörper erst mit einer Schicht aus reinem Bindemittel und dann mit einem Gemisch, wie es oben unter 1.2 beschrieben wurde, überziehen. Zur Erhöhung des Carbonatapatitgehalts an der Oberfläche kann das beschichtete Implantat vor dem Abhärten des Bindemittels mit Carbonatapatit bestreut oder in Carbonatapatit gewälzt werden.

Implantatkörper aus anderen Materialien können auch durch sogenanntes Sputern oder Kathodenzerstäubungsverfahren, das Plasmaspritzverfahren und isostatisches Pressen mit dem erfindungsgemäßen Mittel beschichtet werden. Eine derartige Beschichtung kann außerdem nach Ionenimplantationsverfahren erfolgen. Beim Sputern wird ein Target aus möglichst reinem Carbonatapatit benutzt. Beim Pressen wird die Schicht erst als Gemisch aus weichem Bindemittel, zum Beispiel Agarose, und feinkörnigem Carbonatapatitpulver oder wäßrigen Lösungen aufgebracht.

Zur Herstellung von mit Carbonatapatit überzogenen Implantaten ist weiterhin die chemische Wandlung von Hydroxylapatit in Carbonatapatit, wie sie oben beschrieben wurde, möglich. Implantate, die aus Hydroxylapatit oder hydroxylhaltigen Materialien hergestellt oder mit solchen beschichtet sind, werden mit dem beschriebenen naßchemischen Verfahren an der Oberfläche mit Carbonatapatit beschichtet.

Man kann die vorgefertigten Implantate im Handel verkaufen.

2.2 Herstellung von Implantaten aus carbonatapatithaltigen Materialien

Sind die mechanischen Anforderungen an ein Knochenimplantat nicht sehr hoch oder soll es im Laufe der Zeit ganz von lebendem Knochen durchwachsen oder ersetzt werden, kann es ganz aus Carbonatapatit oder carbonatapatithaltigem Material hergestellt werden.

Funktion

Wie 2.1, zusätzlich kann durch entsprechende Wahl des Bindemittels und/oder der Prorosität vollständiges oder teilweises Durchwachsen oder Ersetzen des Implantats durch lebenden Knochen erreicht werden.

Beispiele

Jede Art von Knochenimplantaten, einschließlich Zahnwurzelimplantate, soweit die mechanische Belastbarkeit ausreicht.

Die Herstellung derartiger Implantate erfolgt aus Carbonatapatit mit Bindemitteln. Die Zusammensetzung des erfindungsgemäßen Mittels ist ähnlich wie oben unter 1.2 beschrieben. Zur Verstärkung können Fasern, Drähte, Gewebe, Geflechte oder Netze eingebracht werden. Diese können beispielsweise aus Metallen, wie Edelmetallen, Stählen oder Titan, oder aus synthetischen und natürlichen Fasern, beispielsweise Kohlenstofffasern, Glasfasern, Whisker, natürlichen und synthetischen Garnen oder aus Tiergeweben gewonnenen Fasern,hergestellt sein. Die Herstellung der Implantate erfolgt in entsprechenden Negativformen und gegebenenfalls durch anschließendes Verdichten unter Anwendung von Druck.

Die folgenden Beispiele erläutern die Erfindung.

**Beispiel 1**

Aus tierischen Knochen wird nach dem in der DE-OS 28 40 064 beschriebenen Verfahren 100 g Hydroxylapatit hergestellt und in einem Becherglas in 1000 ml 1-molarer Natriumcarbonatlösung bei 70°C 100 Minuten gerührt. Der Bodensatz wird danach in üblicher Weise abgefiltert, einmal in 0,5 l 0,001 N Salzsäure und dreimal in destilliertem Wasser gespült und abfiltriert.

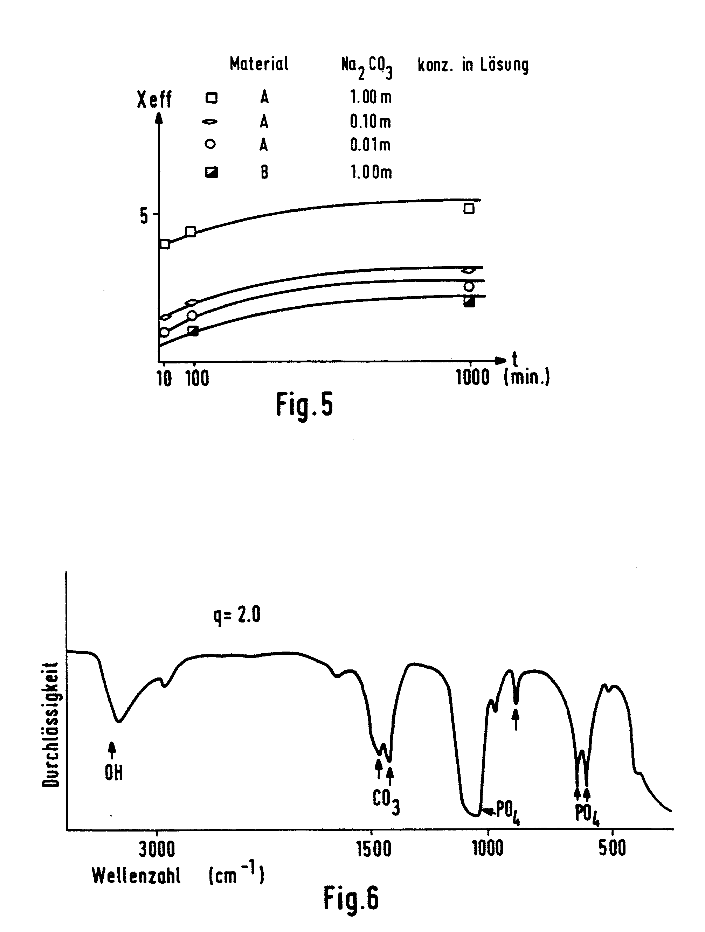

Schließlich wird das Produkt 60 Minuten im Heißluftofen getrocknet. Wie die Figur 5 zeigt, ist schon bei Raumtemperatur nach 100 Minuten in 1-molarer Lösung eine Sättigung von 80% erreicht. Das erhaltene weißliche Material besteht aus feindispersen carbonathaltigen Apatitkristallen, die als Ausgangsprodukt verwendet werden.

**Beispiel 2**

In ein von unten mit Kohlendioxidgas durchblasenes Gefäß, gefüllt mit 2 l 1-molarer Kaliumcarbonatlösung, werden bei Raumtemperatur 500 g Hydroxylapatit (Osbonite[R], Calcitite[R]) zugesetzt. Das eingeblasene Gas bewirkt ein ständiges Durchrühren und hält die Lösung mit Carbonationen gesättigt.

Nach 60 Minuten wird das Material entnommen und wie in Beispiel 1 beschrieben gespült und getrocknet. Das erhaltene Material besteht aus Partikeln, deren Außenschichten in Carbonatapatit umgewandelt wurden und das als Grundmaterial für das Mittel dient.

**Beispiel 3**

In 2 l siedendem destillierten Wasser werden 1 Mol Kaliumphosphat und 2 Mol Kaliumcarbonat gelöst und mit Kaliumhydroxyd auf einen pH von 13,0 eingestellt. Zu der Lösung werden 2 Mol Calciumnitrat zugegeben, und unter ständigem Rühren wird die siedende Mischung 60 Minuten unter Rückfluß gekocht. Danach läßt man die Lösung 24 Stunden bei 60 °C stehen, bevor der Niederschlag abfiltriert, mehrfach mit destilliertem Wasser gespült und 4 Stunden im Heißluftofen bei 120 °C getrocknet wird.

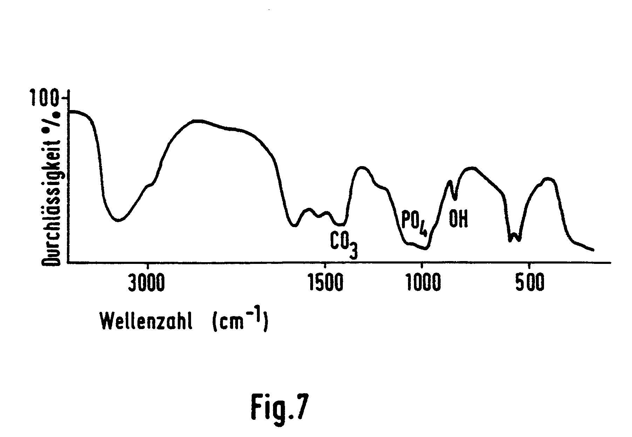

Figur 6 zeigt das Infrarotspektogramm des Produkts, das, wie der Vergleich mit dem Spektogramm von Säugetierknochen in Figur 7 zeigt, dem Knochen chemisch sehr ähnlich ist. Man erhält ca. 250 g Carbonatapatitkristalle, die als Ausgangsprodukt für das Mittel und die Knochenkontaktschichten dienen.

## Beispiel 4

In 2 1 siedendem destillierten Wasser werden 1 Mol Kaliumphosphat und 0,2 Mol Natriumcarbonat gelöst, das weitere Vorgehen erfolgt wie in Beispiel 3. Als Produkt erhält man ca. 250 g carbonathaltige Apatitkristalle, die auch in ihrem Natriumgehalt dem Knochenmineral sehr ähnlich sind und als Ausgangsmaterial für das Mittel und die Kontaktbeschichtung geeignet sind.

## Beispiel 5

In einen 5-1-Stahldruckbehälter werden 1 kg Hydroxylapatitpulver und 500 g Trockeneis eingefüllt, der Behälter verschlossen, auf 600 °C erhitzt und der Druck mit einem entsprechenden Regelventil auf 30 bar eingestellt. Nach 12 Stunden wird der Behälter auf Raumtemperatur abgekühlt, geöffnet und das Carbonatapatitpulver entnommen.

Nach dem Trocknen des Pulvers über 6 Stunden im Heißluftofen bei 150 °C erhält man ca. 1 kg reines Carbonatapatitpulver, das als Ausgangsmaterial für das Mittel dient.

## Beispiel 6

Von nach Beispiel 1 oder einem anderen Verfahren hergestelltem carbonathaltigem Apatit oder Carbonatapatit werden 100 g in 100 ml einer 3%igen Agaroselösung eingerührt. Die weitere Verarbeitung und Anwendung erfolgt nach der in der DE-OS 26 57 370 genauer beschriebenen Methode.

Man erhält ein pastöses Material, das zur Füllung von Knochendefekten geeignet ist.

## Beispiel 7

Das nach Beispiel 2 erzeugte Granulat wird mit etwa gleichem Volumen physiologischer Kochsalzlösung versetzt, so daß es gerade nicht zum Überstehen von Flüssigkeit kommt. Dieses Gemisch wird in Spritzen oder andere Gefäße abgefüllt, luftdicht verschlossen und steril verpackt.

Das so erhaltene Mittel kann während einer Operation direkt in Knochendefekte eingespritzt oder eingefüllt werden.

## Beispiel 8

Das nach Beispiel 4 hergestellte Pulver wird mit gleichen Gewichtsteilen Knochenzementpolymer vermischt und abgepackt. Das Zusetzen des flüssigen Monomeren erfolgt erst bei der Anwendung nach den Vorschriften des Zementherstellers.

Das zunächst pastöse Gemisch kann in Knochen- oder Zahndefekte eingebracht werden und bildet nach kurzer Zeit ein hartes, tragfähiges Material.

## Beispiel 9

Ein Knochenimplantat wird mit dem in der DE-OS 28 40 064 beschriebenen Verfahren mit einer hydroxylapatithaltigen Keramikbeschichtung versehen. Das Implantat wird dann für 24 Stunden in einer 1-molaren Natriumcarbonatlösung bei 70 °C eingelegt.

Nach dieser Behandlung besteht die Kontaktschicht aus carbonathaltigem Apatit.

## Beispiel 10

Ein in einer Sputeranlage (Kathodenzerstäubungsanlage) in seiner Längsachse rotierendes Zahnwurzelimplantat aus Aluminiumoxid wird mit Partikeln, die aus einem Carbonatapatittarget zerstäubt wurden, beschichtet. Durch die extrem hohe Auftreffgeschwindigkeit dringen die Partikel in die Oberfläche des Implantats ein und haften fest auf diesem. Nach 8 Stunden ist eine geschlossene Beschichtung erreicht.

Wie das Ergebnis der ESCA-Untersuchung (Elektronen-Spektroskopie für Chemische Analysen) zeigt, sind an der Oberfläche eines so beschichteten Aluminiumoxidkörpers keine Aluminiumatome mehr nachzuweisen.

## Beispiel 11

Der Schaftteil einer Hüftgelenksendoprothese aus Edelstahl wird mit dünnflüssigem Knochenzement (Methacrylat) bestrichen und mit nach Beispiel 4 hergestelltem carbonathaltigem Apatitpulver bestreut. Nach dem Abbinden des Zements wird der Vorgang wiederholt, bis eine aureichend starke, geschlossene Beschichtung erreicht ist.

## Beispiel 12

Ein Gemisch aus 50% Carbonatapatit (nach Beispiel 3), 48,5 % Wasser und 1,5% Agarose wird angeteigt und auf 100 °C erhitzt. In die Negativform für ein Zahnwurzelimplantat wird dieses Gemisch vor dem Abkühlen auf die Geliertemperatur des Agars eingefüllt. Nach dem Erkalten wird das vorgeformte Implantat entnommen und 72 Stunden an Luft bei Raumtemperatur getrocknet. Anschließend wird der Körper nach dem isostatischen Preßverfahren hoch verdichtet.

Das so erhaltene Implantat kann direkt als Zahnwurzelimplantat verwendet werden.

**Beispiel 13**

Ein Knochentumor im Oberarmknochen wird chirurgisch ausgeräumt und mit dem in Beispiel 8 beschriebenen Material gefüllt. Nach Aushärtung des Zements ist das Füllmaterial sofort mechanisch belastbar und kann einen Teil der Knochenbelastung mittragen, dennoch wird es im Laufe der Zeit von lebendem Knochen durchwachsen und ersetzt.

**Beispiel 14**

Im Gefolge eines Schienbeinbruches kommt es zu einer Knocheneiterung. Der Herd wird eröffnet, nach Ausräumung des abgestorbenen Knochenmaterials wird der Defekt mit Füllmaterial nach Beispiel 7, das je nach Erregerspektrum mit einem Antibiotikum oder kolloidalem Silber versetzt wird, ausgefüllt.

**Beispiel 15**

Bei zu niedrigem Kieferkamm im zahnlosen Kiefer, der einer Vollprothese keinen ausreichenden Halt mehr bietet, wird der Knochen operativ freigelegt, im Frontzahnbereich in Transversalebene durchtrennt, angehoben und mit einem Granulat nach Beispiel 3, versetzt mit etwa gleichem Volumen Frischblut des Patienten, unterfüttert.

Nach dem Wundverschluß wird der so aufgebaute Kieferkamm mit einer provisorischen Kunststoffschiene gestützt, nach 3 Wochen kann die endgültige Versorgung mit einer Zahnprothese erfolgen.

**Beispiel 16**

Bei einem Schenkelhalsbruch eines alten Patienten wird nach Entfernung des Gelenkkopfes und entsprechender Bohrung im Oberschenkelknochen eine wie in Beispiel 11 behandelte Endoprothese zementlos eingesetzt (vgl. Figur 4).

Das Implantat ist sofort belastbar und wächst schnell und fest im Knochen an.

**Beispiel 17**

Nach Extraktion eines nicht mehr erhaltbaren Zahnes wird das Zahnwurzelfach im Kieferknochen mit entsprechenden Bohrern ausgeweitet und eine künstliche Zahnwurzel, beschichtet wie in Beispiel 10 beschrieben, implantiert. Nach einer Einheilphase von einigen Wochen wird auf diese die Suprastruktur (Krone) aufgebracht.

**Beispiel 18**

Bei tiefer Zahnkaries, die bis in den Zahnnerv (Pulpa) reicht, wird das kariöse Material vollständig in üblicher Weise entfernt und anschließend der Defekt mit einer Paste, hergestellt aus 10% Calciumhydroxyd, 40% Carbonatapatit, hergestellt nach Beispiel 5, und 50% physiologischer Kochsalzlösung, gefüllt (sogenannte Nervüberkappung).

**Beispiel 19**

Bei einem marktoten Zahn wird der Wurzelkanal sorgfältig ausgeräumt und der Defekt mit einem pastösen Gemisch aus 65% PMMA-Kunststoff und 35% Carbonatapatitpulver nach Beispiel 1 ausgefüllt (sogenannte Wurzelfüllung).

**Ansprüche**

1. Mittel zur Füllung von Knochen- und Zahndefekten, zum Knochenaufbau, für Knochenkontaktschichten und für Knochen- und Zahnwurzelersatz, dadurch **gekennzeichnet,** daß es Carbonatapatit der Formel I

$$Ca_{10-a-b-x}Me_a^{II}\ Me_b^{I}\ (PO_4)_{6-x}(CO_3)_x\ (OH)_{2-b-x}\cdot(H_2O)_w\quad (I)$$

worin

a   die Anzahl der zweiwertigen Metallionen außer Ca bedeutet und für einen Wert von 0 bis 4,0 steht,

b   die Anzahl der einwertigen Metallionen bedeutet und für einen Wert von 0,05 bis 1,0 steht,

w   die Anzahl der in das Kristallgitter integrierten Wassermoleküle bedeutet und für 0 bis 1,8 steht,

x   die Anzahl der Carbonationen bedeutet und für einen Wert von 0,2 bis 2 steht,

gegebenenfalls zusammen mit körperverträglichen Zusatzstoffen und/oder Verdünnungsmitteln enthält.

2. Mittel nach Anspruch 1, dadurch **gekennzeichnet,** daß es als körperverträgliche Zusatzstoffe organische und/oder anorganische Bindemittel, physiologische Salzlösungen und/oder Medikamente und/oder Hormone enthält.

3. Mittel nach einem der Ansprüche 1 oder 2, dadurch **gekennzeichnet**, daß es 1 bis 100 Gew.-% Carbonatapatit enthält.

4. Mittel nach mindestens einem der Ansprüche 1, 2 oder 3, dadurch **gekennzeichnet**, daß es Carbonatapatit der Formel

$$Ca_{10-x}(PO_4)_{6-x}(CO_3)_x(OH)_{2-x}$$

enthält, worin x für 0,2 bis 2 steht.

5. Verwendung von Carbonatapatit der Formel I

$$Ca_{10-a-b-x}Me_a^{II}\ Me_b^{I}\ (PO_4)_{6-x}(CO_3)_x\ (OH)_{2-b-x}\text{-}(H_2O)_w\quad(I)$$

worin

   a   die Anzahl der zweiwertigen Metallionen außer Ca bedeutet und für einen Wert von 0 bis 4,0 steht,

   b   die Anzahl der einwertigen Metallionen bedeutet und für einen Wert von 0,05 bis 1,0 steht,

   w   die Anzahl der in das Kristallgitter integrierten Wassermoleküle bedeutet und für 0 bis 1,8 steht,

   x   die Anzahl der Carbonationen bedeutet und für einen Wert von 0,2 bis 2 steht,

zur Füllung von Knochen- und Zahndefekten, zum Knochenaufbau, für Knochenkontaktschichten, als Knochenersatz und als Zahnwurzelersatz.

6. Verwendung nach Anspruch 5, dadurch **gekennzeichnet,** daß Carbonatapatit der Formel

$$Ca_{10-x}(PO_4)_{6-x}(CO_3)_x(OH)_{2-x}$$

worin x für 0,2 bis 2 steht, verwendet wird.

7. Implantatkörper, dadurch **gekennzeichnet,** daß er vollständig oder teilweise aus Carbonatapatit besteht oder daß er aus einem für Implantatkörper bekannten Material besteht und vollständig oder teilweise mit einer Schicht aus Carbonatapatit der Formel I

$$Ca_{10-a-b-x}Me_a^{II}\ Me_b^{I}\ (PO_4)_{6-x}(CO_3)_x\ (OH)_{2-b-x}\text{-}(H_2O)_w\quad(I)$$

worin

   a   die Anzahl der zweiwertigen Metallionen außer Ca bedeutet und für einen Wert von 0 bis 4,0 steht,

   b   die Anzahl der einwertigen Metallionen bedeutet und für einen Wert von 0,05 bis 1,0 steht,

   w   die Anzahl der in das Kristallgitter integrierten Wassermoleküle bedeutet und für 0 bis 1,8 steht,

   x   die Anzahl der Carbonationen bedeutet und für einen Wert von 0,2 bis 2 steht,

überzogen ist.

**Claims**

1. A means for filling bone and tooth defects, for bone reconstruction, for bone contact layers and for bone and dental-root replacement, characterised in that it contains carbonate-apatite having the formula I

$$Ca_{10-a-b-x}Me_a^{II}\ Me_b^{I}\ (PO_4)_{6-x}(CO_3)_x\ (OH)_{2-b-x}\text{-}(H_2O)_w\quad(I)$$

in which

   a   stands for the number of divalent metal ions apart from Ca and is equal to 0 to 4.0,

   b   stands for the number of monovalent metal ions and is equal to 0.05 to 1.0,

   w   stands for the number of water molecules incorporated in the crystal lattice and is equal to 0 to 1.8 and

   x   stands for the number of carbonate ions and is equal to 0.2 to 2,

together if required with additives and/or diluents tolerated by the body.

2. A means according to claim 1, characterised in that the tolerated additives therein are organic and/or inorganic binders, physiological salt solutions and/or drugs and/or hormones.

3. A means according to claim 1 or 2, characterised in that it contains from 1 to 100 wt.% carbonate-apatite.

4. A means according to at least one of claims 1, 2 or 3, characterised in that it contains carbonate-apatite having the formula

$$Ca_{10-x}(PO_4)_{6-x}(CO_3)_x(OH)_{2-x}$$

where x stands for 0.2 to 2.

5. Use of carbonate-apatite having the formula I

$$Ca_{10-a-b-x}Me_a^{II}\ Me_b^{I}\ (PO_4)_{6-x}(CO_3)_x\ (OH)_{2-b-x}\text{-}(H_2O)_w\quad(I)$$

in which

a stands for the number of divalent metal ions apart from Ca and is equal to 0 to 4.0,

b stands for the number of monovalent metal ions and is equal to 0.05 to 1.0,

w stands for the number of water molecules incorporated in the crystal lattice and is equal to 0 to 1.8, and

x stands for the number of carbonate ions and is equal to 0.2 to 2,

for filling bone and tooth defects, for bone reconstruction, for bone contact layers, as a bone replacement and as a tooth-root replacement.

6. Use according to claim 5, characterised in that carbonate-apatite having the formula

$$Ca_{10-x}(PO_4)_{6-x}(CO_3)_x(OH)_{2-x}$$

in which x stands for 0.2 to 2, is used.

7. An implant member characterised in that it consists completely or partly of carbonate-apatite or it consists of a material of known use for implant members and is completely or partly coated with a layer of carbonate apatite having the formula I

$$Ca_{10-a-b-x}Me_a^{II}\ Me_b^{I}\ (PO_4)_{6-x}(CO_3)_x\ (OH)_{2-b-x}\text{-}(H_2O)_w\quad (I)$$

in which

a stands for the number of divalent metal ions apart from Ca and is equal to 0 to 4.0,

b stands for the number of monovalent metal ions and is equal to 0.05 to 1.0,

w stands for the number of water molecules incorporated in the crystal lattice and is equal to 0 to 1.8, and

x stands for the number of carbonate ions and is equal to 0.2 to 2.

**Revendications**

1. Matériau d'obturation des défauts osseux et dentaires, pour la constitution des os, pour les couches de contact des os et pour le remplacement des os et des racines dentaires, caractérisé en ce qu'il contient de la carbonate-apatite de formule I

$$Ca_{10-a-b-x}Me_a^{II}\ Me_b^{I}\ (PO_4)_{6-x}(CO_3)_x\ (OH)_{2-b-x}\text{-}(H_2O)_w\quad (I)$$

dans laquelle

a est le nombre des ions métalliques divalents autres que Ca et représente une valeur de 0 à 4,0,

b est le nombre des ions métalliques monovalents et représente une valeur de 0,05 à 1,0,

w est le nombre des molécules d'eau intégrées dans le réseau cristallin et est compris entre 0 et 1,8.

x est le nombre des ions carbonate et représente une valeur de 0,2 à 2,

et éventuellement des additifs et/ou diluants compatibles avec l'organisme.

2. Matériau selon la revendication 1, caractérisé en ce qu'il contient comme additifs compatibles avec l'organisme des liants organiques et/ou inorganiques, des solutions salines physiologiques et/ou des médicaments et/ou des hormones.

3. Matériau selon l'une des revendications 1 ou 2, caractérisé en ce qu'il contient 1 à 100% en poids de carbonate-apatite.

4. Matériau selon au moins l'une des revendications 1, 2 ou 3, caractérisé en ce qu'il contient une carbonate-apatite de formule :

$$Ca_{10-x}(PO_4)_{6-x}(CO_3)_x(OH)_{2-x}$$

dans laquelle x est compris entre 0,2 et 2.

5. Utilisation d'une carbonate-apatite de formule I :

$$Ca_{10-a-b-x}Me_a^{II}\ Me_b^{I}\ (PO_4)_{6-x}(CO_3)_x\ (OH)_{2-b-x}\text{-}(H_2O)_w\quad (I)$$

dans laquelle

a est le nombre des ions métalliques divalents autres que Ca et représente une valeur de 0 à 4,0,

b est le nombre des ions métalliques monovalents et représente une valeur de 0,05 à 1,0,

w est le nombre des molécules d'eau intégrées dans réseau cristallin et est compris entre 0 et 1,8,

x est le nombre des ions carbonate et représente une valeur de 0,2 à 2,

pour l'obturation des défauts osseux et dentaires, pour la constitution des os, pour les couches de contact des os, pour le remplacement des os et pour le remplacement des racines dentaires.

6. Utilisation selon la revendication 5, caractérisée en ce que l'on utilise une carbonate-apatite de formule :

$$Ca_{10-x}(PO_4)_{6-x}(CO_3)_x(OH)_{2-x}$$

dans laquelle x est compris entre 0,2 et 2.

7. Implant caractérisé en ce qu'il consiste totalement ou partiellement en carbonate-apatite ou en ce qu'il consiste en un matériau connu pour les implants et en ce qu'il est revêtu totalement ou partiellement d'une couche de carbonate-apatite de formule I :

$$Ca_{10-a-b-x}Me_a^{II} \quad Me_b^{I} \quad (PO_4)_{6-x}(CO_3)_x \quad (OH)_{2-b-x^-}$$
$$(H_2O)_w \quad (I)$$

dans laquelle

a    est le nombre des ions métalliques divalents autres que Ca et représente une valeur de 0 à 4,0,

b    est le nombre des ions métalliques monovalents et représente une valeur de 0,05 à 1,0,

w    est le nombre des molécules d'eau intégrées dans le réseau cristallin et est compris entre 0 et 1,8,

x    est le nombre des ions carbonate et représente une valeur de 0,2 à 2.

Fig.1

Fig.2

Fig.3

EP 0 193 588 B1

Fig. 4

**Fig.5**

**Fig.6**

Fig.7